# EUROPEAN PATENT SPECIFICATION

(11) **EP 2 902 015 B1**
(45) Date of publication and mention of the grant of the patent: **04.12.2019**
(21) Application number: 13842023.7
(22) Date of filing: 28.09.2013
(51) Int. Cl.: A61K 9/20, A61K 9/48, A61K 9/16, A61K 31/165, A61K 47/32, A61P 25/24

(54) **PREPARATION METHOD OF AGOMELATINE SOLID PREPARATION**
VERFAHREN ZUR HERSTELLUNG EINER FESTEN AGOMELATINZUBEREITUNG
PROCÉDÉ DE PRÉPARATION D'UNE PRÉPARATION SOLIDE D'AGOMÉLATINE

(30) Priority: 28.09.2012 CN 201210381283
(43) Date of publication of application: 05.08.2015
(73) Proprietor: Zhejiang Huahai Pharmaceutical Co., Ltd, Zhejiang 317024 (CN)
(72) Inventor: PENG, Junqing, Linhai Zhejiang 317024 (CN); XU, Biao, Linhai Zhejiang 317024 (CN); CAO, Yu, Linhai Zhejiang 317024 (CN); CHEN, Liang, Linhai Zhejiang 317024 (CN); YANG, Zhuan, Linhai Zhejiang 317024 (CN); YU, Hui, Linhai Zhejiang 317024 (CN); LI, Yanyan, Linhai Zhejiang 317024 (CN)
(74) Representative: Pfenning, Meinig & Partner mbB
(86) International application number: PCT/CN2013/084552
(87) International publication number: WO 2014/048380

(56) References cited:
- CN-A- 102 670 514
- CN-A- 102 988 315
- US-A1- 2009 069 434
- REN, XINGFA: 'Preparation of Azithromycin Solid Dispersion' CNKI CHINA MASTER'S S THESES FULL-TEXT DATABASE 30 April 2009, page 10, XP008175334

## Description

### Field of the Invention

The present invention relates to the field of medical technology, and in particular relates to a preparation method of agomelatine solid preparation.

### Background of the Invention

Agomelatine tablet, sold under the trade name of Valdoxan®/Thymanax® at a strength of 25mg, is developed by Servier, a French company. Agomelatine is mainly used for the treatment of depression in adults. It is approved for marketing in EU in February 2009, as the first antidepressant of melatonin receptor agonist in the world. The molecular structural formula of agomelatine is as follows:

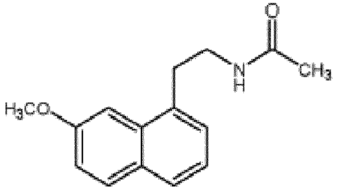

Agomelatine is nearly insoluble in hydrochloric acid solution of pH 2.0, acetate buffer solution of pH 4.5, phosphate buffer solution of pH 6.8 and water. Therefore, the dissolution of agomelatine needs to be enhanced in preparation process, so as to enhance its bioavailability.

There are technical difficulties in the development of preparation for medicament having poor dissolubility. The main diffculties are hardly absorbing or bioavailability decreasing caused by low dissolution. Therefore, the problem of dissolution of a medicament having poor dissolubility needs to be adressed.

Patent No. CN102670514A discloses a method for preparing agomelatine by a hot melt extrusion process.

Patent No. CN102218050A discloses a method for preparing agomelatine tablets. The agomelatine is micronized to enhance the dissolution, especially is micronized to a smaller particle size to achieve a good effect of dissolution. However, the micronizing of active pharmaceutical ingredients (API) requires specialized airflow crusher equipment, and involves complex production procedure. The particle size of the API needs to be controlled. All of these decrease the production efficiency.

### Summary of the Invention

The present invention provides a preparation method of agomelatine solid preparation, comprising: firstly preparing a composition of agomelatine and cross-linked polyvinylpolypyrrolidone by hot-melt granulation technology, then sieving the obtained composition and mixing it with a pharmaceutically acceptable carrier, and tableting or encapsulating wherein the process of heating to melt for granulation is achieved by using an oven or a high-shear mixer granulator with a jacket.

Cross-linked polyvinylpolypyrrolidone (PVPP) is white to milk white, finely dispersed, free flowing, almost tasteless, odourless or slightly odorous powder. The main component of cross-linked polyvinylpolypyrrolidone is homopolymer of 1-ethenyl-2-pyrrolidone. Different types of commercial cross-linked polyvinylpolypyrrolidone, such as the cross-linked polyvinylpolypyrrolidone of Kollidon CL, Kollidon CL-M, Polyplasdone XL, and Polyplasdone XL-10, can be selected in accordance with the present invention.

In general, amorphous form of an API has better solubility than crystalline form of the same API. A preparation comprising amorphous API usually has a better dissolution rate than a preparation comprising crystalline of the same API. However, amorphous state is a higher energy state, and is often transformed into crystalline state during the preparing and storing of a preparation, so that the dissolution rate of the medicament decreases, and the bioavailability of the medicament is affected.

In the present invention, agomelatine and cross-linked polyvinylpolypyrrolidone are mixed and heated, so that agomelatine is melted and dispersed in the form of fluid into the pores of the cross-linked polyvinylpolypyrrolidone. Then the resultant is cooled and sieved. Since agomelatine is adsorbed by the cross-linked polyvinylpolypyrrolidone after being melted, the intermolecular distance increases, and amorphous form of agomelatine is obtained after cooling. Furthermore, the crystallization of agomelatine is inhibited due to the separation effect of the network molecular structure of the cross-linked polyvinylpolypyrrolidone. Therefore, agomelatine will not be transformed into crystalline state during the preparing and storing of the preparation thereof, so that stability of crystal form of the product is maintained, and in turn the dissolution rate and bioavailability of the product are ensured.

After agomelatine and the cross-linked polyvinylpolypyrrolidone are melted and granulated, an agomelatine composition can be easily obtained by sieving without the step of crushing, because the particles are relatively loose. The composition has good flowability, which is convenient for sequential preparation processing.

The ratio of PVPP and agomelatine is screened in the present invention. It is found that agomelatine can not be present in amorphous state in the resultant composition when the propotion of the cross-linked polyvinylpolypyrrolidone is too low. It can be ensured that agomelatine exists in amorphous state when the ratio of the cross-linked polyvinylpolypyrrolidone to agomelatine is more than 2:1. The API has good solubility under this ratio. The solubility is improved over the API in crystalline form. However, if the ratio of the cross-linked polyvinylpolypyrrolidone to agomelatine is too high, the weight of prepared tablet or capsule is relatively high. Therefore, according to the present invention, the weight ratio of the cross-linked polyvinylpolypyrrolidone to agomelatine is from 2:1 to 10:1, preferably from 2:1 to 5:1. Under this ratio, the tablet or capsule has better quality and appropriate weight.

Since agomelatine has various crystal forms, such as crystal form I with a melting point of about 98°C, crystal form II with a melting point of about 108°C, and crystal form VI with a melting point of about 94°C, in the context, heating the agomelatine to melt means heating the material to a temperature higher than the melting point of agomelatine, for example, to 95°C, 100°C, 120°C or 150°C, depending on the crystal forms of agomelatine. Specific temperature should be selected depending on the crystal forms of the API, as long as the API can be melted at this temperature.

There is no strict requirement for the particle size of agomelatine, because the API is to be melted in the invention. This is relatively simple for the process of preparation, without the need of mechanical pulverizing or airflow crushing the agomelatine, saving the cost and production time.

The agomelatine compositions prepared according to the present invention are placed in an environment at a temperature of 40 ± 2°C and a relative humidity of 75 ± 5% for 6 months. The spectrum of X-ray diffraction shows that these compositions maintain the amorphous state after 6 months, which indicates that the form of the composition is stable.

According to the present invention, the pharmaceutically acceptable additives include diluents, adhesives, lubricant, etc. The prepared composition is mixed with the excipients listed below, and then tableted or encapsulated to provide the solid preparation described above after further processing.

The diluents suitable for the agomelatine solid preparation of the invention include: mannitol, lactose, microcrystalline cellulose, starch, corn starch, partially pregelatinized starch, saccharose, lactose, glucose, dextrin, calcium hydrophosphate, calcium dihydrogen phosphate, and maltitol.

The adhesives suitable for the agomelatine solid preparation of the invention include: hydroxypropyl cellulose, povidone, copolyvidone, and hypromellose.

The lubricants suitable for the agomelatine solid preparation of the invention include: talcum powder, magnesium stearate, sodium stearyl fumarate, and aerosil.

The agomelatine composition provided by the present invention has stable quality. The obtained solid preparation has good *in vitro* dissolution property. And the method for preparing the composition is simple and clean, and is suitable for industrial production.

### Description of Drawings

Figure 1 is the X-ray powder diffraction spectrum of the raw material of agomelatine.
Figure 2 is the X-ray powder diffraction spectrum of the agomelatine composition prepared by example 2.
Figure 3 is the X-ray powder diffraction spectrum of the agomelatine composition prepared by example 2 after being placed at 40°C and 75% humidity for 6 months.

### Detailed description of the invention

The invention is further illustrated below in details in combination with examples, and is not limited to the examples described below. The "%" used herein refers to "wt%". For the purpose of proving the advantages of the invention, the agomelatine used in examples 1 to 8 have substantially similar particle size.

### Example 1

### Preparation of the agomelatine composition:

10g of agomelatine of crystal form II and 20g of cross-linked polyvinylpolypyrrolidone (type: Kollidon CL) were weighted and mixed evenly, then placed in an oven of 160°C for 10 min, and taken out for sieving and cooling.

### Example 2

### Preparation of the agomelatine composition:

100g of agomelatine of crystal form VI and 300g of cross-linked polyvinylpolypyrrolidone (type: Kollidon CL-M) were weighted and granulated in a granulator with a jacket at a temperature of 105°C, stirred for 5 min, and then taken out for sieving and cooling.

### Example 3

### Preparation of the agomelatine composition:

10g of agomelatine of crystal form II and 40g of cross-linked polyvinylpolypyrrolidone (type: Polyplasdone XL) were weighted and mixed evenly, then placed in an oven of 140°C for 10 min, and taken out for sieving and cooling.

### Example 4

### Preparation of the agomelatine composition:

50g of agomelatine of crystal form I and 250g of cross-linked polyvinylpolypyrrolidone (type: Polyplasdone XL-10) were weighted and granulated in a granulator with a jacket at a temperature of 105°C, stirred for 5 min, and then taken out for sieving and cooling.

### Example 5

### Preparation of the agomelatine composition:

10g of agomelatine of crystal form I and 100g of cross-linked polyvinylpolypyrrolidone (type: Kollidon CL) were weighted and mixed evenly, then placed in an oven of 160°C for 10 min, and then taken out for sieving and cooling.

### Example 6

Preparation of tablets and capsules of agomelatine. The formulations were as follows:

| Components | Formulation 1 | Formulation 2 | Formulation 3 | Formulation 4 | Formulation 5 |
|---|---|---|---|---|---|
| | Dosage per tablet mg | Dosage per tablet mg | Dosage per tablet mg | Dosage per tablet mg | Dosage per tablet mg |
| Agomelatine | 25 | 25 | 25 | 25 | 25 |
| Cross-linked polyvinylpolypyrrolidone | 50 | 75 | 100 | 125 | 250 |
| lactose | 44 | 19 | - | - | - |
| pregelatinized starch | - | - | - | 28 | - |
| microcrystalline cellulose | | - | 23.5 | - | 190 |
| hydroxypropylcellulose | | 10 | - | - | - |
| copolyvidone | 10 | - | - | - | 30 |
| povidone | - | - | - | 20 | - |
| magnesium stearate | 1 | 1 | 1.5 | 2 | 5 |
| in total | 130 | 130 | 150 | 200 | 500 |

Preparation process of agomelatine tablets/capsules: agomelatine and cross-linked polyvinylpolypyrrolidone were weighted, mixed evenly, and placed in an oven for 10 min, or agomelatine and cross-linked polyvinylpolypyrrolidone were hot-melt granulated in a granulator with a jacket. Then the resultant was taken out, sieved, cooled, and mixed with other adjuvants for tableting or encapsulating (formulations 1, 2, 3 were tableted, and formulations 4, 5 were encapsulated).

### Comparative Example 7

The materials of formulation 1 were directly mixed and tableted according to the ratio in the formulation.

### Comparative Example 8

The materials of formulation 5 were directly mixed and encapsulated according to the ratio in the formulation.

### Example 9

### Test for dissolution rate of the solid preparation:

Tablets and capsules were test by stirring paddle method in a dissolution media of pH = 2.0, at rotate speed of 50rpm, and in 1000ml to provide a curve of dissolution rate. The results are shown below:

| Formulation | 15min | 30min | 45min | 60min |
|---|---|---|---|---|
| Formulation 1 | 78 | 91.5 | 96.4 | 98 |
| Formulation 2 | 78 | 94.1 | 99.3 | 98.3 |
| Formulation 3 | 78 | 90.9 | 98 | 97 |
| Formulation 4 | 80.9 | 88 | 94 | 96.4 |
| Formulation 5 | 73.6 | 87.1 | 98.3 | 100 |
| Comparative Example 7 | 50 | 63 | 74 | 82 |
| Comparative Example 8 | 45 | 66 | 76 | 85 |

### Example 10

### Investigation of the stability of agomelatine composition

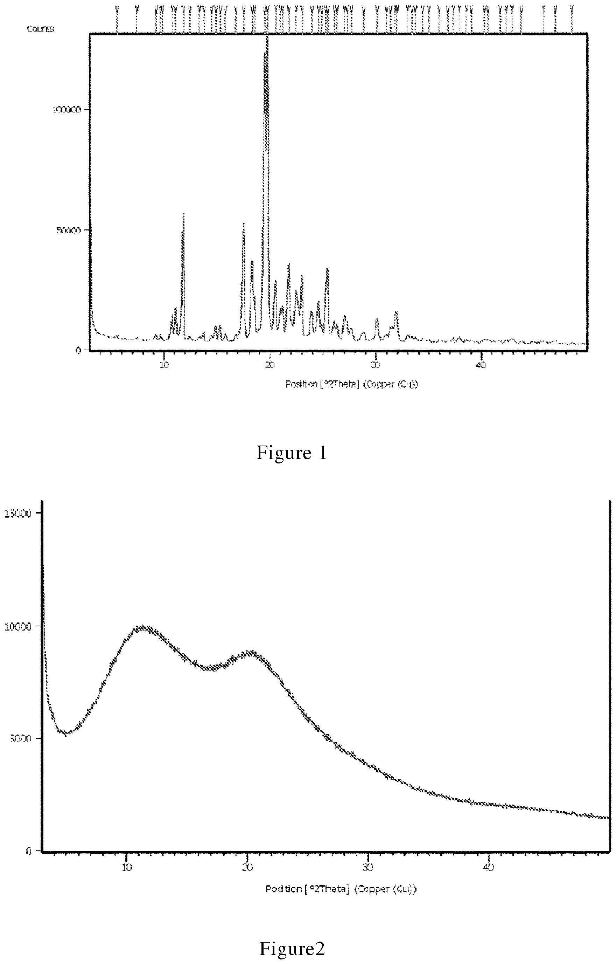

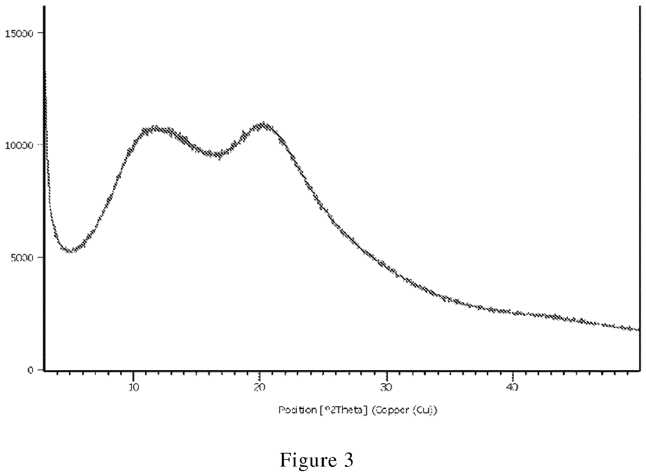

The X-ray powder diffraction spectrums of raw material of agomelatine, agomelatine composition prepared by example 2, and the agomelatine composition after being placed for 6 months were shown in figure1, 2 and 3, respectively.

## Claims

1. A preparing method of an agomelatine solid preparation, comprising the steps of:
a. mixing agomelatine and cross-linked polyvinylpolypyrrolidone to provide a mixture a;
b. heating the mixture a to melt the agomelatine, and cooling to provide composition b;
c. mixing composition b and pharmaceutically acceptable carriers to obtain mixed particles c; and
d. tableting or encapsulating the mixed particles c,
**characterized in that** the process of heating to melt for granulating in step b. is achieved by using an oven or a high-shear mixer granulator with a jacket.

2. The preparing method according to claim 1, **characterized in that** the weight ratio of cross-linked polyvinylpolypyrrolidone and agomelatine is from 2:1 to 10:1.

3. The preparing method according to claim 2, **characterized in that** the weight ratio of cross-linked polyvinylpolypyrrolidone and agomelatine is from 2:1 to 5:1.

4. The preparing method according to claim 1, **characterized in that** the pharmaceutically acceptable carriers include diluent, disintegrant, adhesive, and lubricant, wherein the diluent is selected form mannitol, lactose, microcrystalline cellulose, starch, corn starch, partially pregelatinized starch, saccharose, lactose, glucose, dextrin, calcium hydrophosphate, calcium dihydrogen phosphate, and maltitol; the adhesive is selected from hydroxypropylcellulose, povidone, copolyvidone, and hypromellose; and the lubricant is selected from talcum powder, magnesium stearate, sodium stearyl fumarate, and aerosil.

## Patentansprüche

1. Herstellungsverfahren für eine Agomelatin-Feststoffpräparation, umfassend die Schritte:
Mischen von Agomelatin und vernetztem Polyvinylpolypyrrolidon, um ein Gemisch a bereitzustellen;
Erwärmen des Gemisches a, um das Agomelatin zu schmelzen, und Abkühlen, um die Zusammensetzung b bereitzustellen;
Mischen der Zusammensetzung b und pharmazeutisch akzeptabler Träger, um gemischte Partikel c zu erhalten; und
Tablettieren oder Verkapseln der gemischten Partikel c,
**dadurch gekennzeichnet, dass** der Prozess des Erwärmens, um zu schmelzen, zum Granulieren in Schritt b. unter Verwendung eines Ofens oder eines Mischergranulators mit hoher Scherkraft mit einem Mantel erreicht wird.

2. Herstellungsverfahren nach Anspruch 1, **dadurch gekennzeichnet, dass** das Gewichtsverhältnis von vernetztem Polyvinylpolypyrrolidon und Agomelatin von 2:1 bis 10:1 ist.

3. Herstellungsverfahren nach Anspruch 2, **dadurch gekennzeichnet, dass** das Gewichtsverhältnis von vernetztem Polyvinylpolypyrrolidon und Agomelatin von 2:1 bis 5:1 ist.

4. Herstellungsverfahren nach Anspruch 1, **dadurch gekennzeichnet, dass** die pharmazeutisch akzeptablen Träger Verdünnungsmittel, Zerfallsmittel, Haftmittel und Schmiermittel enthalten, wobei das Verdünnungsmittel ausgewählt ist aus Mannitol, Laktose, mikrokristalliner Cellulose, Stärke, Maisstärke, teilweise vorgelatinierter Stärke, Saccharose, Laktose, Glucose, Dextrin, Calciumhydrophosphat, Calciumdihydrogenphosphat und Maltit; das Haftmittel ausgewählt ist aus Hydroxypropylcellulose, Povidon, Copolyvidon und Hypromellose; und das Schmiermittel ausgewählt ist aus Talkumpulver, Magnesiumstearat, Natriumstearylfumarat und Aerosil.

## Revendications

1. Procédé de préparation d'une préparation solide d'agomélatine, comprenant les étapes de :
a. mélange d'agomélatine et de polyvinylpolypyrrolidone réticulée pour obtenir un mélange a ;
b. chauffage du mélange a pour faire fondre l'agomélatine, et refroidissement pour obtenir la composition b ;
c. mélange de la composition b et de véhicules pharmaceutiquement acceptables pour obtenir des particules mixtes c ; et
d. compression ou encapsulation des particules mixtes c,
**caractérisé en ce que** le processus de chauffage de fusion pour granulation dans l'étape b. est conduit en utilisant un four ou un mélangeur-granulateur à cisaillement élevé avec une chemise.

2. Procédé de préparation selon la revendication 1, **caractérisé en ce que** le rapport en poids de polyvinylpolypyrrolidone réticulée et d'agomélatine est de 2:1 à 10:1.

3. Procédé de préparation selon la revendication 2, **caractérisé en ce que** le rapport en poids de polyvinylpolypyrrolidone réticulée et d'agomélatine est de 2:1 à 5:1.

4. Procédé de préparation selon la revendication 1, **caractérisé en ce que** les véhicules pharmaceutiquement acceptables comprennent un diluant, un délitant, un adhésif et un lubrifiant, le diluant étant choisi parmi le mannitol, le lactose, la cellulose microcristalline, l'amidon, l'amidon de maïs, l'amidon partiellement prégélatinisé, le saccharose, le lactose, le glucose, la dextrine, l'hydrophosphate de calcium, le dihydrogénophosphate de calcium et le maltitol ; l'adhésif est choisi parmi l'hydroxypropylcellulose, la povidone, la copolyvidone et l'hypromellose ; et le lubrifiant est choisi parmi la poudre de talc, le stéarate de magnésium, le fumarate de stéaryle de sodium et Aerosil.
